(19) Europäisches Patentamt
European Patent Office
Office européen des brevets

(11) Veröffentlichungsnummer: **0 157 324**
**B1**

(12) # EUROPÄISCHE PATENTSCHRIFT

(45) Veröffentlichungstag der Patentschrift: **31.08.88**

(21) Anmeldenummer: **85103503.0**

(22) Anmeldetag: **25.03.85**

(51) Int. Cl.⁴: **C 07 D 495/04**, C 07 D 211/90, A 61 K 31/44 // C07D409/04, C07C153/017, (C07D495/04, 333:00, 221:00)

(54) **Tetrahydrothienopyridine, Verfahren zu ihrer Herstellung sowie ihre Verwendung als Arzneimittel.**

(30) Priorität: **06.04.84 DE 3412947**

(43) Veröffentlichungstag der Anmeldung: **09.10.85 Patentblatt 85/41**

(45) Bekanntmachung des Hinweises auf die Patenterteilung: **31.08.88 Patentblatt 88/35**

(84) Benannte Vertragsstaaten: **AT BE CH DE FR GB IT LI NL SE**

(56) Entgegenhaltungen:
**EP-A-0 111 453**
**EP-A-0 111 455**
**US-A-4 284 634**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Goldmann, Siegfried, Dr., Am Osterholz 91, D-5600 Wuppertal 11 (DE)**
Erfinder: **Schramm, Matthias, Dr., Paffrather Strasse 38, D-5000 Köln 80 (DE)**
Erfinder: **Thomas, Günter, Dr., Claudiusweg 9, D-5600 Wuppertal 1 (DE)**
Erfinder: **Gross, Rainer, Dr., Platzhoffstrasse 23, D-5600 Wuppertal 1 (DE)**

EP 0 157 324 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Tetrahydrothienopyridine, ein Verfahren zu ihrer Herstellung sowie ihre Verwendung als Arzneimittel insbesondere als kreislaufbeeinflussende Arzneimittel.

Aus dem US-Patent Nr. 4 284 634 sind bereits Dihydropyridin-Derivate bekannt, deren Substituenten in 2- und 3-Position gegebenenfalls gemeinsam Sauerstoff oder Stickstoff enthaltende Ringe bilden. Die erfindungsgemäßen Thienopyridine werden in dieser Vorpublikation nicht erwähnt und durch sie auch nicht nahegelegt. Die Anmeldung EP-A-0 111 455 beschreibt Dihydropyridinlactone, die sich ebenfalls von den erfindungsgemäßen Thieno-Verbindungen eindeutig unterscheiden.

Die neuen Verbindungen sind durch folgende allgemeine Formel (I) gekennzeichnet

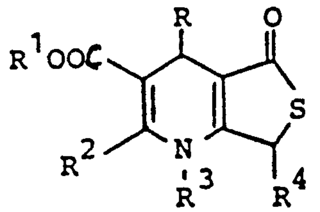

(I)

in welcher

R für einen Phenyl-, Naphthyl, Thienyl-, Furyl-, Pyrryl-, Pyrazolyl-, Imidazolyl-, Oxazolyl-, Isoxazolyl-, Thiazolyl-, Pyridyl-, Pyridazinyl-, Pyrimidyl-, Pyrazinyl-, Chinolyl-, Isochinolyl-, Indolyl-, Benzimidazolyl-, Chinazolyl-, Chinoxalyl-, Thionaphthenyl-, Isothionaphthenyl-, Chromonyl-, Thiochromonyl-, Chromenyl-, Thiochromenyl-, Benzoxadiazolyl- oder Benzthiadiazolylrest steht wobei die genannten Reste gegebenenfalls 1 bis 3 gleiche oder verschiedene Substituenten aus der Gruppe Alkyl (1 bis 20 Kohlenstoffatome), Alkenyl (bis zu 20 Kohlenstoffatome), Alkinyl (bis zu 20 C-Atome), Alkoxy (1 bis 20 C-Atome), Fluor, Chlor, Brom, Iod, Trifluormethyl, Monofluoralkoxy (1 bis 10 C-Atome), Polyfluoralkoxy (1 bis 10 6-Atome), Hydroxy, Amino, Monoalkylamino- (1 bis 10 C-Atome), Dialkylamino (1 bis 10 C-Atome), Nitro, Cyano, Azido, Carboxy, Carbonamido, Sulfonamido, $SO_m$-Alkyl ($m = 0$ bis 2, 1 bis 20 C-Atome), Phenyl, Benzyl, Benzyloxy oder Benzylthio enthalten können wobei die vier letztgenannten Substituenten gegebenenfalls 1 bis 3 Reste aus der Gruppe Alkyl (1 bis 5 C-Atome), Alkoxy (1 bis 5 C-Atome), Alkylthio (1 bis 5 C-Atome), Fluor, Chlor, Brom, Iod, Cyano, Nitro, Azido, Hydroxy, Trifluormethyl, Amino, Alkylamino (1 bis 6 C-Atome) oder Dialkylamino (je 1 bis 6 C-Atome) enthalten können,

$R^1$ für einen geradkettigen, verzweigten oder cyclischen gesättigten oder ungesättigten Kohlenwasserstoffrest (bis zu 20 C-Atome) steht, der gegebenenfalls durch 1 oder 2 nicht benachbarte Sauerstoff- oder Schwefelatome in der Kette unterbrochen sein kann und der gegebenenfalls substituiert sein kann durch ein oder mehrere Fluoratome, $NO_2$, Trialkylsilyl (3 bis 12 C-Atome), -OH, -Cl, Br, J, -CN, Amino, Alkylamino (1 bis 10 C-Atome), Dialkylamino (je 1 bis 10 C-Atome), Benzylalkylamino (Alkylrest mit 1 bis 8 C-Atomen), Phenyl, Naphthyl oder Pyridyl,

$R^2$

für Wasserstoff, $-NH_2$, -CHO, -CN, $CH_2OH$ oder einen geradkettigen oder verzweigten, gesättigten oder ungesättigten Kohlenwasserstoffrest steht (bis zu 8 C-Atome),

$R^3$ für Wasserstoff oder einen geradkettigen oder verzweigten Alkylrest (1 bis 10 C-Atome) steht, der gegebenenfalls durch ein oder zwei nicht benachbarte Sauerstoffatome in der Alkylkette unterbrochen ist und der gegebenenfalls substituiert sein kann durch Fluor, Chlor, Brom, Iod, -CN, $NH_2$ -OH oder Morpholino und

$R^4$ für Wasserstoff oder einen geradkettigen oder verzweigten Alkylrest (bis zu 10 C-Atome) steht

in Form von Isomeren, Isomerengemischen, Racematen, optische Antipoden sowie ihre pharmazeutisch unbedenklichen Salze.

Als Salze seien beispielsweise genannt Hydrochloride, Hydrogensulfate, Sulfate, Hydrogenphosphate, Acetate, Maleate, Benzoate, Citronate, Tartrate oder Lactate.

Von besonderem Interesse sind Verbindungen der allgemeinen Formel (I), in welcher

R für einen Phenyl-, Naphthyl-, Thienyl-, Pyrryl-, Imidazolyl-, Oxazolyl-, Isoxazolyl-, Thiazolyl-, Pyridyl-, Pyridazinyl-, Pyrimidyl-, Chinolyl-, Isochinolyl-, Indolyl -, Benzimidazolyl-, Chinazolyl-, Thionaphthenyl-, Chromonyl-, Thiochromonvl-, Chromonyl-, Thiochromonyl-, Benzoxadiazolyl- oder Benzthiadiazolylrest steht, wobei die genannten Reste gegebenenflals 1 bis 3 gleiche oder verschiedene Substituenten aus der Gruppe Alkyl (1 bis 15 Kohlenstoffatome), Alkenyl (bis zu 15 Kohlenstoffatome), Alkinyl (bis zu 15 C-Atome), Alkoxy (1 bis 15 C-Atome), Fluor, Chlor, Brom, Iod, Trifluormethyl, Monofluoralkoxy (1 bis 5 C-Atome), Polyfluoralkoxy (1 bis 5 C-Atome), Hydroxy-amino, Monoalkylamino (1 bis 5 C-Atome), Dialkylamino (1 bis 5 C-Atome), Nitro, Cyano, Azido, Carboxy, Carbonamido, Sulfonamido, $SO_m$-Alkyl (m = 0 bis 2, 1 bis 10 C-Atome), Phenyl, Benzyl, Benzyloxy oder Benzylthio enthalten können wobei die vier letztgenannten Substituenten gegebenenfalls 1 bis 3 Reste aus der Gruppe Alkyl (1 bis 4 C-Atome), Alkoxy (1 bis 4 C-Atome), Alkylthio (1 bis 4 C-Atome), Fluor, Chlor, Cyano, Nitro, Hydroxy, Trifluormethyl-, Amino, Alkylamino (1 bis 4 C-Atome) oder Dialkylamino (1 bis 4 C-Atome) enthalten können,

$R^1$ für einen geradkettigen, verzweigten oder cyclischen gesättigten oder ungesättigten Kohlenwasserstoffrest (bis zu 15 C-Atome) darstellt, der gegebenenfalls durch 1 oder 2 nicht benachbarte Sauerstoff-oder Schwefelatome in der Kette unterbrochen sein kann und der gegebenenfalls substituiert sein kann durch ein oder mehrere Fluoratome, $NO_2$, Trialkylsilyl (3 bis 9 C-Atome), -OH, -Cl, -CN, Amino, Alkylamino (1 bis 5 C-Atome) oder Dialkylamino (je 1 bis 5 C-Atome),

$R^2$ für Wasserstoff, -CHO, -CN oder einen geradkettigen oder verzweigten, gesättigten oder ungesättigten Kohlenwasserstoffrest steht (bis zu 6 C-Atome),

$R^3$ für Wasserstoff oder einen geradkettigen oder verzweigten Alkylrest (bis zu 6 C-Atome) steht, der gegebenenfalls durch ein oder zwei nicht benachbarte Sauerstoffatome in der Alkylkette unterbrochen ist und der gegebenenfalls substituiert sein kann durch Fluor, Chlor, -CM, -OH oder Morpholino und

$R^4$ für Wasserstoff oder einen geradkettigen oder verzweigten Alkylrest (bis zu 6 C-Atome) steht.

Vorzugsweise seien solche Verbindungen der allgemeinen Formel (I) genannt, in welcher

R für einen Phenyl-, Naphthyl-, Thienyl-, Oxazolyl-, Isoxazolyl-, Thiazolyl-, Pyridyl-, Pyrimidyl-, Chinolyl-, Isochinolyl-, Indolyl-, Benzimidazolyl-, Chinazolyl-, Thionaphthenyl-, Chromonyl-, Thiochromonyl-, Chromenyl-, Thiochromenyl-, Benzoxadiazolyl- oder Benzthiadiazolylrest steht wobei die genannten Reste gegebenenfalls 1 bis 2 gleiche oder verschiedene Substituenten aus der Gruppe Alkyl (1 bis 10 Kohlenstoffatome), Alkenyl (bis zu 10 Kohlenstoffatome), Alkoxy (1 bis 10 C-Atome), Fluor, Chlor, Brom, Trifluormethyl, Monofluoralkoxy (1 bis 5 C-Atome), Polyfluoralkoxy (1 bis 5 C-Atome), Hydroxy, Monoalkylamino (1 bis 5 C-Atome), Dialkylamino (1 bis 5 C-Atome), Nitro, Cyano, Azido, $SO_m$-Alkyl (m = 0 bis 2, 1 bis 5 C-Atome), Phenyl, Benzyl, Benzyloxy- oder Benzylthio enthalten können wobei die vier letztgenannten Substituenten gegebenenfalls 1 bis 3 Reste aus der Gruppe Alkyl (1 bis 3 C-Atome), Alkoxy (1 bis 3 C-Atome), Alkylthio (1 bis 3 C-Atome), Fluor, Chlor, Cyano, Nitro, Trifluormethyl, enthalten können,

$R^1$ für einen geradkettigen, verzweigten oder cyclischen, gesättigten oder ungesättigten Kohlenwasserstoffrest (bis zu 10 C-Atome) steht, der gegebenenfalls durch 1 oder 2 nicht benachbarte Sauerstoff- oder Schwefelatome in der Kette unterbrochen sein kann und der gegebenenfalls substituiert sein kann durch ein oder mehrere Fluoratome, $NO_2$, Trialkylsilyl (3 bis 6 C-Atome), -OH, -Cl, -CN, Amino, Alkylamino (1 bis 3 C-Atome) oder Dialkylamino (je 1 bis 3 C-Atome),

$R^2$ für Wasserstoff oder einen geradkettigen oder verzweigten, gesättigten oder ungesättigten Kohlenwasserstoffrest steht (bis zu 4 C-Atome),

$R^3$ für Wasserstoff oder einen geradkettigen oder verzweigten Alkylrest (bis zu 4 C-Atome) steht, der gegebenenfalls durch ein oder zwei nicht benachbarte Sauerstoffatome in der Alkylkette unterbrochen ist und der gegebenenfalls substituiert sein kann durch Fluor, Chlor, -OH oder Morpholino und

$R^4$ für Wasserstoff oder einen geradkettigen Alkylrest (1 bis 4 C-Atome) steht.

Die erfindungsgemäßen Verbindungen der allgemeinen Formel (I) können hergestellt werden, indem man Benzylidenver bindungen der allgemeinem Formel (II)

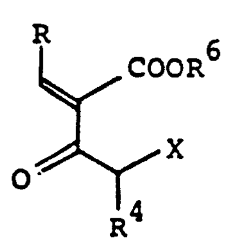

(II)

in welcher

R und $R^4$ die oben angegebene Bedeutung haben,

X für Halogen steht und

$R^6$ für einen Alkylrest mit bis zu 6 Kohlenstoffatomen steht, mit Thiolaten der allgemeinen Formel (III)

Me-S-$R^5$ (III)

in welcher

Me ein Alkali bedeutet und

$R^5$ für eine Thiolschutzgruppe steht,

in Gegenwart von inerten organischen Lösungsmitteln bei Temperaturen von -60 bis +80° unter Abspaltung von Alkalihalogenid (MeX) zu Verbindungen der allgemeinen Formel (IV)

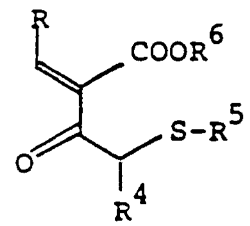

(IV)

in welcher

R, $R^4$, $R^5$ und $R^6$ die oben angegebene Bedeutung haben,

umsetzt und anschließend die Verbindung der Formel (IV) mit Aminocrotonsäureestern der allgemeinen Formel (V)

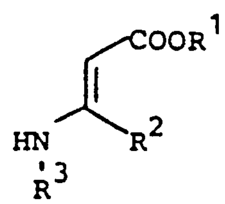

(V)

in welcher $R^1$, $R^2$ und $R^3$ die oben angegebene Bedeutung haben, in Gegenwart von inerten organischen Lösungsmitteln zu Dihydropyridin der allgemeinen Formel (VI)

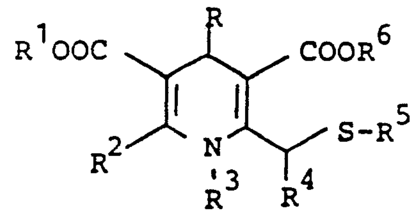

(VI)

in welcher

R, $R^1$, $R^2$, $R^3$, $R^4$, $R^5$ und $R^6$ die oben angegebene Bedeutung haben, umsetzt

und dieses Dihydropyridin der Formel (VI) anschließend unter Abspaltung der Thiolschutzgruppe ($R^5$) zu Verbindungen der allgemeinen Formel (I) lactonisiert.

Die Reaktionsschritte des erfindungsgemäßen Verfahrens können sowohl als Eintopfreaktion ohne Isolierung der hierbei entstehenden Zwischenprodukte (IV) und (VI) oder in getrennten Reaktionsstufen unter Isolierung der Zwischenstufen (IV) und (VI) durchgeführt werden.

Als Thioschutzgruppen ($R^5$) seien vorzugsweise Acylgruppen mit bis zu 8 Kohlenstoffatomen oder tertiäre Alkylgruppen, insbesondere tertiär-Butyl genannt.

Als Alkali (Me) seien vorzugsweise Lithium, Natrium und Kalium genannt.

Als Lösungsmittel für die Umsetzung von (II) nach (IV) seien vorzugsweise niedere Alkohole mit bis zu 6 Kohlenstoffatomen genannt.

Als Halogen (X) seien vorzugsweise Chlor und Brom genannt.

Die Abspaltung der Thiolschutzgruppe aus Verbindungen der allgemeinen Formel (VI) erfolgt vorzugsweise mit geeigneten Abspaltungsagentien, wie z.B. organischen und anorganischen Säuren.

Die Einführung der Thiolgruppe (S-$R^5$) erfolgt vorzugsweise bei Temperaturen zwischen -20 und +60°C, insbesondere zwischen 0° und 30°C.

Die Benzylidenverbindungen der allgemeinen Formel (II) sind bekannt bzw. können nach bekannten Methoden hergestellt werden (vgl.: Surrey et al. JACS 66, 1933 (1944)).

Die zur Herstellung verwendeten Schwefelverbindungen der allgemeinen Formel (III) sind bekannt oder können nach bekannten Methoden hergestellt werden (vgl. C.Ulrich, Ann. 109 (1958), 272).

Die bei der Herstellung verwendeten Aminocrotonsäureester der allgemeinen Formel (V) sind ebenfalls bekannt oder können nach bekannten Methoden hergestellt werden (vgl.: A. C. Cope, J. Am. Chem. Soc. 67, 1017 (1945)).

Außer den Verbindungen der Herstellungsbeispiele seien folgende erfindungsgemäße Verbindungen beispielhaft genannt:

1) 4(2-Benzylthiophenyl)-2-methyl-5-oxo-1,4,5,7-te-tetrahydrothieno [3-4-b]pyridin-3-carbonsäureetyl-ester

2) 4-(2-Benzyloxyphenyl)-2-methyl-5-oxo-1,4,5,7-tetrahydrothieno [3,4-b]pyridin-3-carbonsäuremethylester

3) 4-(3-Chlorphenyl)-2-methyl-5-oxo-1,4,5,7-tetrahy-drothieno [3,4-b] pyridin-3-carbonsäurebutylester

4) 2-Cyano-4-(2-methylphenyl)-5-oxo-1,4,5,7-tetrahy-drothieno [3,4-b]pyridin-3-carbonsäureethylester

5) 2-Methyl-5-oxo-4-(4-oxo-2-phenyl-4H-chromen-8-yl)-1,4,5,7-tetrahydrothieno [3,4-b]pyridin-3-carbonsäure-ethylester

6) 2-Methyl-4-(2-[4-methylbenzyl]thiophenyl)-5-oxo 1,4,5,7-tetrahydrothieno [3,4-b]pyridin-3-carbonsäu-reethylester

7) 2-Methyl-5-oxo-4-(2-trifluormethylphenyl)-1,4,5,7-tetrahydrothieno [3,4-b] pyridin-3-carbonsäure (N-benzyl-N-methyl)-aminoethylester

Die erfindungsgemäßen Verbindungen zeigen ein nicht Vorhersehbares und wertvolles pharmakologisches Wirkungsspektrum. Sie können als Cardiotonika zur Verbesserung der Herzkontraktilität dienen. Darüber hinaus können sie dadurch, daß sie den Ca++-Einstrom in die Zelle erhöhen, als Antihypotonica, zur Senkung von Blutzucker, zur Abschwellung von Schleimhäuten und zur Beeinflussung des Salz- und/oder Flüssigkeitshaushaltes eingesetzt werden.

Die erfindungsgemäßen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerten, nichttoxischer, pharmazeutisch geeigneter Trägerstoffe oder

Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermitteln (z. B. Polyoxyethylen-Fettsäure-Ester, Polyoxyethylen-Fettalkohol-Ether, Alkylsulfonate und Arylsulfonate), Dispergiermittel (z. B. Lignin-Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,0001 bis 1 mg/kg, vorzugsweise etwa 0,001 bis 0,5 mg/kg Körpergewicht täglich zur Erzielung wirksamer Ergebnisse zu verabreichen, und bei oraler Applikation beträgt die Dosierung etwa 0,01 bis 20 mg/kg, vorzugsweise 0,1 10 bis 10 mg/kg Körpergewicht täglich.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation, größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Ausführungsbeispiele**

**Beispiel 1**

2-Methyl-5-oxo-4-(2-trifluormethylphenyl)-1,4,5,7-tetrahydrothieno [3,4-6] pyridin-3-carbonsäureethylester

a)

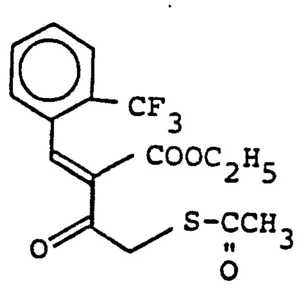

50 mmol 4-Chlor-2-(2-trifluormethylbenzyliden)-3-ketobuttersäureethylester (E/Z-Gemisch) werden in 100 ml Ethanol gelöst und bei 0°C mit 50 mmol Kaliumthioacetat versetzt, bei Raumtemperatur 2 Stunden gerührt, eingeengt, in $CH_2Cl_2$ aufgenommen, mit Wasser gewaschen und getrocknet. Es verbleibt 4-Acylthio-2-(2-trifluor-methylbenzyliden)-3-ketobuttersäureethylester (E/Z-Gemisch) als Öl.

b)

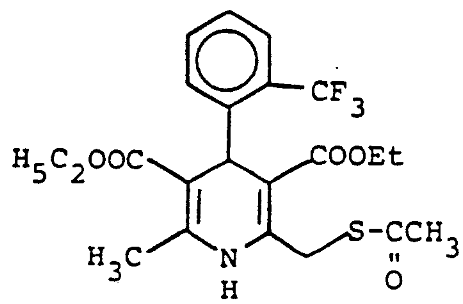

Das oben erhaltene Öl wird mit 50 mmol 3-Aminocroton-säureethylester in 50 ml Ethanol über Nacht am Rückfluß gekocht, eingeengt und an Kieselgel mit Toluol/ Essigester (8:2) chromatographiert.

Es verbleibt 2-Acylthiomethyl-6-methyl-4-(2-trifluormethylphenyl)-1, 4-dihydropyridin-3, 5-dicarbonsäurediethylester.

MS (471, M⊕), 326 (100 %), 284 (40 %), 238 (20 %)

c)

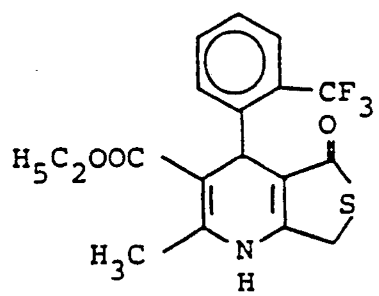

10 mmol 2-Acylthiomethyl-6-methyl-4-(2-trifluormethyl-phenyl)-1, 4-dihydropyridin-3, 5-dicarbonsäurediethylester werden in 40 ml Ethanol gelöst mit 6 ml gesättigter ethanolischer HCl 2 Stunden gekocht, mit Wasser gefällt und anschließend aus Ethanol umkristallisiert.
Fp.: 213-216°C

**Beispiel 2** (Herstellung analog Beispiel 1)

2-Methyl-5-oxo-4-(4-oxo-2-phenyl-4H-thiochromen-8-yl)-1,4,5,7-tetrahydrothieno [3,4-b]pyridin-3-carbonsäureethylester

a)

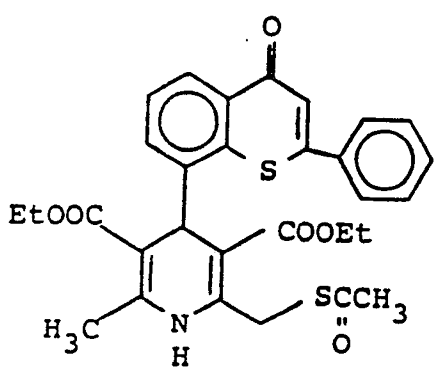

Darstellung analog Beispiel 1, Fp.: 120°C.

b)

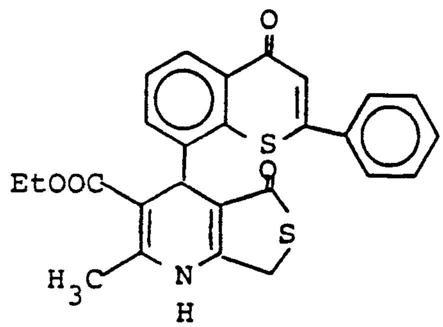

Darstellung analog Beispiel 1, Fp.: < 270°C
1H-NMR (DMSO): 0,8 (3H,t), 2,4 (s, 3H), 3,9 (q, 2H), 4,1 und 4,3 (2d, je 1H), 5,4 (s, 1H), 7,25 (s, 1H), 7,6 (m, 5H), 7,9 (m, 2H), 8,3 (dd, 1H), 10,2 (s, NH)

**Beispiel 3**

2-Methyl-5-oxo-4-(4-oxo-2-phenyl-4-H-thiochromen-8-yl)-1,4,5,7-tetrahydrothieno [3,4-b]pyridin-3-carbonsäurebutylester
Darstellung analog Beispiel 2, jedoch ohne Isolierung von Zwischenstufen.
Fp.: 210 bis 220°C.

**Beispiel 4**

2-Methyl-5-oxo-4-(2-methylphenyl)-1,4,5,7-tetrahydro-thieno [3,4-6]pyridin-3-carbonsäuremethylester
Darstellung analog Beispiel 3, Fp.: 240 bis 43°C.

**Beispiel 5**

2-Methyl-5-oxo-4-(2-methylphenyl)-1,4,5,7-tetrahydrothieno[3,4-b]pyridin-3-carbonsäurebutylester
Darstellung analog Beispiel 3, Fp.: 160 bis 163°C.

**Beispiel 6**

4-(2-Chlorphenyl)-2-methyl-5-oxo-1,4,5,7-tetrahydro-thieno [3,4-b]pyridin-3-carbonsäureethylester
Darstellung analog Beispiel 3, Fp.: 234 bis 237°C.

**Beispiel 7**

2-Methyl-5-oxo-4-(4-oxo-2-phenyl-4H-thiochromen-8-yl)-1,4,5,7-tetrahydrothieno [3,4-b] pyridin-3-carbonsäuremethylester
Darstellung analog Beispiel 3, Fp.: < 270°C.
MS: 461 (M+, 30 %), 402 (30 %), 238 (80 %), 224 (50 %), 44 (100 %).

**Patentansprüche**

1. Tetrahydrothienopyridine der allgemeinen Formel (I)

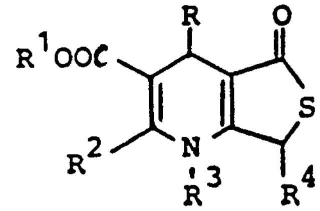

(I)

in welcher

R für einen Phenyl-, Naphthyl-, Thienyl-, Furyl-, Pyrryl-, Pyrazolyl-, Imidazolyl-, Oxazolyl-, Isoxazolyl-, Thiazolyl-, Pyridyl-, Pyridazinyl-, Pyrimidyl-, Pyrazinyl-, Chinolyl-, Isochinolyl-, Indolyl-, Benzimidazolyl-, Chinazolyl-, Chinoxalyl-, Thionaphthenyl-, Isothionaphthenyl-, Chromonyl-, Thiochromonyl-, Chromenyl-, Thiochromenyl-, Benzoxadiazolyl- oder Benzthiadiazolylrest steht wobei die genannten Reste gegebenenfalls 1 bis 3 gleiche oder verschiedene Substituenten aus der Gruppe Alkyl (1 bis 20 Kohlenstoffatome), Alkenyl (bis zu 20 Kohlenstoffatome), Alkinyl (bis zu 20 C-Atome), Alkoxy (1 bis 20 C-Atome), Fluor, Chlor, Brom, Iod, Trifluormethyl, Monofluoralkoxy (1 bis 10 C-Atome), Polyfluoralkoxy (1 bis 10 C-Atome), Hydroxy, Amino, Monoalkylamino- (1 bis 10 C-Atome), Dialkylamino (1 bis 10 C-Atome), Nitro, Cyano, Azido, Carboxy, Carbonamido, Sulfonamido, $SO_m$-Alkyl (m = 0 bis 2, 1 bis 20 C-Atome), Phenyl, Benzyl, Benzyloxy oder Benzylthio enthalten können wobei die vier letztgenannten Substituenten gegebenenfalls 1 bis 3 Reste aus der Gruppe Alkyl (1 bis 5 C-Atome), Alkoxy (1 bis 5 C-Atome), Alkylthio (1 bis 5 C-Atome), Fluor, Chlor, Brom, Iod, Cyano, Nitro, Azido, Hydroxy, Trifluormethyl, Amino, Alkylamino (1 bis 6 C-Atome) oder Dialkylamino (je 1 bis 6 C-Atome) enthalten können,

$R^1$ für einen geradkettigen, verzweigten oder cyclischen gesättigten oder ungesättigten Kohlenwasserstoffrest (bis zu 20 C-Atome) steht, der gegebenenfalls durch 1 oder 2 nicht benachbarte Sauerstoff- oder Schwefelatome in der Kette unterbrochen sein kann und der gegebenenfalls substituiert sein kann durch ein oder mehrere Fluoratome, $NO_2$ Trialkylsilyl (3 bis 12 C-Atome), -OH, -Cl, Br, J, -CN, Amino, Alkylamino (1 bis 10 C-Atome), Dialkylamino (je 1 bis 10 C-Atome), Benzylalkylamino (Alkylrest mit 1 bis 8 C-Atomen), Phenyl, Naphthyl oder Pyridyl,

$R^2$ für Wasserstoff, $NH_2$, -CHO, -CN, $-CH_2OH$ oder einen geradkettigen oder verzweigten, gesättigten oder ungesättigten Kohlenwasserstoffrest steht (bis zu 8 C-Atome),

$R^3$ für Wasserstoff oder einen geradkettigen oder verzweigten Alkylrest (1 bis 10 C-Atome) steht, der gegebenenfalls durch ein oder zwei nicht benachbarte Sauerstoffatome in der Alkylkette unterbrochen ist und der gegebenenfalls substituiert sein kann durch Fluor, Chlor, Brom, Iod, -CN, $-NH_2$, -OH oder Morpholino und

$R^4$ für Wasserstoff oder einen geradkettigen oder verzweigten Alkylrest (bis zu 10 C-Atome) steht
sowie ihre pharmazeutisch unbedenklichen Salze.

2. Verbindungen der allgemeinen Formel (I) gemäß Anspruch 1
in welcher

R für einen Phenyl-, Naphthyl-, Thienyl-, Pyrryl-, Imidazolyl-, Oxazolyl-, Isoxazolyl-, Thiazolyl-, Pyridyl-, Pyridazinyl-, Pyrimidyl-, Chinolyl-, Isochinolyl-, Indolyl-, Benzimidazolyl-, Chinazolyl-, Thionaphthenyl-, Chromonyl-, Thiochromonyl-, Chromenyl-, Thiochromenyl-, Benzoxadiazolyl- oder Benzthiadiazolylrest steht wobei die genannten Reste gegebenenfalls 1 bis 3 gleiche oder verschiedene Substituenten aus der Gruppe Alkyl (1 bis 15 Kohlenstoffatome), Alkenyl (bis zu 15 Kohlenstoffatome), Alkinyl (bis zu 15 C-Atome), Alkoxy (1 bis 15 C-Atome), Fluor, Chlor, Brom, Iod, Trifluormethyl, Monofluoralkoxy (1 bis 5 C-Atome), Polyfluoralkoxy (1 bis 5 C-Atome), Hydroxy-amino, Monoalkylamino (1 bis 5 C-Atome), Dialkylamino (1 bis 5 C-Atome), Nitro, Cyano, Azido, Carboxy, Carbonamido, Sulfonamido, $SO_m$-Alkyl (m = 0 bis 2, 1 bis 10 C-Atome), Phenyl, Benzyl, Benzyloxy oder Benzylthio enthalten können wobei die vier letztgenannten Substituenten gegebenenfalls 1 bis 3 Reste aus der Gruppe Alkyl (1 bis 4 C-Atome), Alkoxy (1 bis 4 C-Atome), Alkylthio (1 bis 4 C-Atome), Fluor, Chlor, Cyano, Nitro, Hydroxy, Trifluormethyl-, Amino, Alkylamino (1 bis 4 C-Atome) oder Dialkylamino (1 bis 4 C-Atome) enthalten können,

$R^1$ für einen geradkettigen, verzweigten oder cyclischen gesättigten oder ungesättigten Kohlenwasserstoffrest (bis zu 15 C-Atome) darstellt, der gegebenenfalls durch 1 oder 2 nicht benachbarte Sauerstoff- oder Schwefelatome in der Kette unterbrochen sein kann und der gegebenenfalls substituiert sein kann durch ein oder mehrere Fluoratome, $NO_2$, Trialkylsilyl (3 bis 9 C-Atome), -OH, -Cl, -CN, Amino, Alkylamino (1 bis 5 C-Atome) oder Dialkylamino (je 1 bis 5 C-Atome),

$R^2$ für Wasserstoff, -CHO, -CN oder einen geradkettigen oder verzweigten, gesättigten oder ungesättigten Kohlenwasserstoffrest steht (bis zu 6 C-Atome),

$R^3$ für Wasserstoff oder einen geradkettigen oder verzweigten Alkylrest (bis zu 6 C-Atome) steht, der gegebenenfalls durch ein oder zwei nicht benachbarte Sauerstoffatome in der Alkylkette unterbrochen ist und der gegebenenfalls substituiert sein kann durch Fluor, Chlor, -CN, -OH oder Morpholino und

$R^4$ für Wasserstoff oder einen geradkettigen oder verzweigten Alkylrest (bis zu 6 C-Atome) steht.

3. Verbindungen der allgemeinen Formel (I) gemäß Anspruch 1 in welcher

R für einen Phenyl-, Naphthyl-, Thienyl-, Oxazolyl-, Isoxazolyl-, Thiazolyl-, Pyridyl-, Pyrimidyl-, Chinolyl-, Isochinolyl-, Indolyl-, Benzimidazolyl-, Chinazolyl-, Thionaphthenyl, Chromonyl-, Thiochromonyl, Chromenyl-, Thiochromenyl-, Benzoxadiazolyl oder Benzthiadiazolylrest steht wobei die genannten Reste gegebenenfalls 1 bis 2 gleiche oder verschiedene Substituenten aus der Gruppe Alkyl (1 bis 10 Kohlenstoffatome), Alkenyl (bis zu 10 Kohlenstoffatome), Alkoxy (1 bis 10 C-Atome), Fluor, Chlor, Brom, Trifluormethyl, Monofluoralkoxy (1 bis 5 C-Atome), Polyfluoralkoxy (1 bis 5 C-Atome), Hydroxy, Monoalkylamino (1 bis 5 C-Atome), Dialkylamino (1 bis 5 C-Atome), Nitro, Cyano, Azido, $SO_m$-Alkyl (m = 0 bis 2, 1 bis 5 C-Atome), Phenyl, Benzyl, Benzyloxy- oder Benzylthio enthalten können wobei die vier letztgenannten Substituenten gegebenenfallls 1 bis 3 Reste aus der Gruppe Alkyl (1 bis 3 C-Atome), Alkoxy (1 bis 3 C-Atome), Alkylthio (1 bis 3 C-Atome), Fluor, Chlor, Cyano, Nitro, Trifluormethyl, enthalten können,

$R^1$ für einen geradkettigen, verzweigten oder cyclischen, gesättigten oder ungesättigten Kohlenwasserstoffrest (bis zu 10 C-Atome) steht, der gegebenenfalls durch 1 oder 2 nicht benachbarte Sauerstoff- oder Schwefelatome in der Kette unterbrochen sein kann und der gegebenenfalls substituiert sein kann durch ein oder mehrere Fluoratome, $NO_2$, Trialkylsilyl (3 bis 6 C-Atome), -OH, -Cl, -CN, Amino, Alkylamino (1 bis 3 C-Atome) oder Dialkylamino (je 1 bis 3 C-Atome),

$R^2$ für Wasserstoff oder einen geradkettigen oder verzweigten, gesättigten oder ungesättigten Kohlenwasserstoffrest steht (bis zu 4 C-Atome),

$R^3$ für Wasserstoff oder einen geradkettigen oder verzweigten Alkylrest (bis zu 4 C-Atome) steht, der gegebenenfalls durch ein oder zwei nicht benachbarte Sauerstoffatome in der Alkylkette unterbrochen ist und der gegebenenfalls substituiert sein kann durch Fluor, Chlor, -OH oder Morpholino und

$R^4$ für Wasserstoff oder einen geradkettigen Alkylrest (1 bis 4 C-Atome) steht.

4. Verfahren zur Herstellung von Verbindungen der allgemeinen Formel (I)

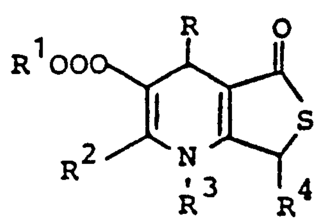

(I)

in welcher

R für einen Phenyl-, Naphthyl-, Thienyl-, Furyl-, Pyrryl-, Pyrazolyl-, Imidazolyl, Oxazolyl-, Isoxazolyl, Thiazolyl-, Pyridyl-, Pyridazinyl-, Pyrimidyl-, Pyrazinyl-, Chinolyl-, Isochinolyl, Indolyl-, Benzimidazolyl, Chinazolyl-, Chinoxalyl-, Thionaphthenyl, Isothionaphthenyl, Chromonyl-, Thiochromonyl, Chromenyl-, Thiochromenyl-, Benzoxadiazolyl- oder Benzthiadiazolylrest steht wobei die genannten Reste gegebenenfalls 1 bis 3 gleiche oder verschiedene Substituenten aus der Gruppe Alkyl (1 bis 20 Kohlenstoffatome), Alkenyl (bis zu 20 Kohlenstoffatome), Alkinyl (bis zu 20 C-Atome), Alkoxy (1 bis 20 C-Atome), Fluor, Chlor, Brom, Iod, Trifluormethyl, Monofluoralkoxy (1 bis 10 C-Atome), Polyfluoralkoxy (1 bis 10 C-Atome), Hydroxy-, Amino, Monoalkylamino (1 bis 10 C-Atome), Dialkylamino (1 bis 10 C-Atome), Nitro, Cyano, Azido, Carboxy, Carbalkoxy, Carbonamido, Sulfonamido, $SO_m$-Alkyl (m = 0 bis 2, 1 bis 20 C-Atome), Phenyl, Benzyl, Benzyloxy oder Benzylthio enthalten können wobei die vier letztgenannten Substituenten gegebenenfalls 1 bis 3 Reste aus der Gruppe Alkyl (1 bis 5 C-Atome), Alkoxy (1 bis 5 C-Atome), Alkylthio (1 bis 5 C-Atome), Fluor, Chlor, Brom, Iod, Cyano, Nitro, Azido, Hydroxy, Trifluormethyl, Amino, Alkylamino (1 bis 6 C-Atome) oder Dialkylamino (je 1 bis 6 C-Atome) enthalten können,

$R^1$ für einen geradkettigen, verzweigten oder cyclischen gesättigten oder ungesättigten Kohlenwasserstoffrest (bis zu 20 C-Atome) steht, der gegebenenfalls durch 1 oder 2 nicht benachbarte Sauerstoff- oder Schwefelatome in der Kette Unterbrochen sein kann und der gegebenenfalls substituiert sein kann durch ein oder mehrere Fluoratome, $NO_2$ Trialkylsilyl (3 bis 12 C-Atome), -OH, -Cl, Br, J, -CM, Amino, Alkylamino (1 bis 10 C-Atome), Dialkylamino (je 1 bis 10 C-Atome), Benzylalkylamino (Alkylrest mit 1 bis 8 C-Atomen), Phenyl, Naphthyl oder Pyridyl,

$R^2$ für Wasserstoff, $-NH_2$, -CHO, -CN, $CH_2OH$ oder einen geradkettigen oder verzweigten, gesättigten oder ungesättigten Kohlenwasserstoffrest steht (bis zu 8 C-Atome),

$R^3$ für Wasserstoff oder einen geradkettigen oder verzweigten Alkylrest (bis zu 10 C-Atome) steht, der gegebenenfalls durch ein oder zwei nicht benachbarte Sauerstoffatome in der Alkylkette unterbrochen ist und der gegebenenfalls substituiert sein kann durch Fluor, Chlor, Brom, Iod, -CN, $NH_2$ -OH oder Morpholino und

$R^4$ für Wasserstoff oder einen geradkettigen oder verzweigten Alkylrest (bis zu 10 C-Atome) steht,

sowie ihre pharmazeutisch unbedenklichen Salze, dadurch gekennzeichnet, daß man Benzylidenverbindungen der allgemeinen Formel (II)

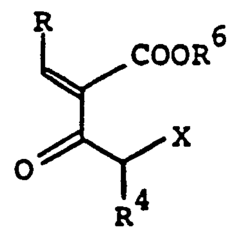

(II)

in welcher

R und $R^4$ die oben angegebene Bedeutung haben,

X für Halogen steht und

$R^6$ für einen Alkylrest mit bis zu 6 Kohlenstoffatomen steht,

mit Thiolaten der allgemeinen Formel (III)

$Me-S-R^5$ (III)

in welcher

Me ein Alkali bedeutet und

$R^5$ für eine Thiolschutzgruppe steht,

in Gegenwart von inerten organischen Lösungsmitteln bei Temperaturen von -60 bis +80°C unter Abspaltung von Alkalihalogenid (MeX) zu Verbindungen der allgemeinen Formel (IV)

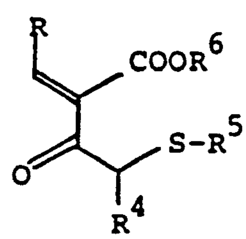

(IV)

in welcher

R, $R^4$, $R^5$ und $R^6$ die oben angegebene Bedeutung haben,

umsetzt und anschließend die Verbindung der Formel (IV) mit Aminocrotonsäureestern der allgemeinen Formel (V)

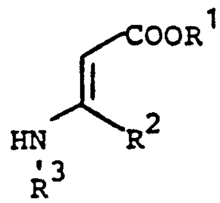

(V)

in welcher

$R^1$, $R^2$ und $R^3$ die oben angegebene Bedeutung haben,

in Gegenwart von inerten organischen Lösungsmitteln zu Dihydropyridin der allgemeinen Formel (VI)

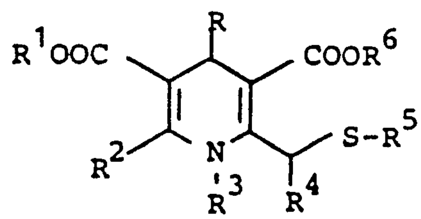

(VI)

in welcher

R, $R^1$, $R^2$, $R^3$, $R^4$, $R^5$ und $R^6$ die oben angegebene Bedeutung haben, umsetzt

und dieses Dihydropyridin der Formel (VI) anschließend unter Abspaltung der Thiolschutzgruppe ($R^5$) zu Verbindungen der allgemeinen Formel (I) lactonisiert.

5. Verfahren gemäß Anspruch 4, dadurch gekennzeichnet, daß man die Umsetzung unter Isolierung der Verbindungen (IV) und (VI) durchführt.
6. Arzneimittel enthaltend mindestens eine Verbindung der allgemeinen Formel (I) gemäß Anspruch 1.
7. Verfahren zur Herstellung von Arzneimitteln, dadurch gekennzeichnet, daß man Verbindungen der allgemeinen Formel (I) gemäß Anspruch 1 gegebenenfalls unter Verwendung von inerten Hilfs- und Trägerstoffen in eine geeignete Applikationsform überführt.
8. Verwendung von Verbindungen der allgemeinen Formel (I) gemäß Anspruch 1 zur Herstellung von Arzneimitteln zur Bekämpfung von Kreislauferkrankungen.
9. Verwendung von Verbindungen der allgemeinen Formel (I) gemäß Anspruch 1 zur Herstellung von Arzneimitteln zur Behandlung von Coronarerkrankungen, Hypertonie, Diabetes und Störungen des Salzhaushaltes.

**Claims**

1. Tetrahydrothienopyridines of the general formula (I)

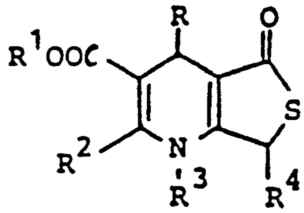

(I)

in which

R represents a phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl, quinoxalyl, thionaphthenyl, isothionaphthenyl, chromonyl,thiochromonyl, chromenyl, thiochromenyl, benzoxadiazolyt or benzothiadiazolyl radical, it being possible for the radicals mentioned optionally to contain 1 to 3 identical or different substituents from the group comprising alkyl (1 to 20 carbon atoms),

alkenyl (up to 20 carbon atoms), alkinyl (up to 20 C- atoms), alkoxy (1 to 20 C-atoms), fluorine, chlorine, bromine, iodine, trifluoromethyl, monofluoroalkoxy (1 to 10 C-atoms), polyfluoroalkoxy (1 to 10 C-atoms), hydroxyl, amino, monoalkylamino (1 to 10 C-atoms), dialkylamino (1 to 10 C-atoms), nitro, cyano, azido, carboxyl, carboxamido, sulphonamido, $SO_m$-alkyl (m = 0 to 2, 1 to 20 C-atoms), phenyl, benzyl, benzyloxy and benzylthio, it being possible for the last four substituents mentioned optionally to contain 1 to 3 radicals from the group comprising alkyl (1 to 5 C-atoms), alkoxy (1 to 5 C-atoms), alkylthio (1 to 5 C-atoms), fluorine, chlorine, bromine, iodine, cyano, nitro, azido, hydroxl, trifluoromethyl, amino, alkylamino (1 to 6 C-atoms) or dialkylamino (in each case 1 to 6 C-atoms),

$R^1$ represents a straight-chain, branched or cyclic saturated or unsaturated hydrocarbon radical (up to 20 C-atoms), which can optionally be interrupted in the chain by 1 or 2 non-adjacent oxygen or sulphur atoms and which can optionally be substituted by one or more fluorine atoms, $NO_2$, trialkylsilyl (3 to 12 C-atoms), -OH, -Cl, Br, I, -CH, amino, alkylamino (1 to 10 C-atoms), dialkylamino (in each case 1 to 10 C-atoms), benzylalkylamino (alkyl radical with 1 to 8 C-atoms), phenyl, naphthyl or pyridyl,

$R^2$ represents hydrogen, $-NH_2$, -CHO, -CH, $CH_2OH$ or a straight-chain or branched, saturated or unsaturated hydrocarbon radical (up to 8 C-atoms),

$R^3$ represents hydrogen or a straight-chain or branched alkyl radical (1 to 10 C-atoms) which is optionally interrupted in the alkyl chain by one or two non-adjacent oxygen atoms and can optionally be substituted by fluorine, chlorine, bromine, iodine, -CN, $-NH_2$, -OH or morpholino, and

$R^4$ represents hydrogen or a straight-chain or branched alkyl radical (up to 10 C-atoms) and their pharmaceutically acceptable salts.

2. Compounds of the general formula (I) according to Claim 1,

in which

R represents a phenyl, naphthyl, thienyl, pyrryl, imidazolyl, oxazolyl, isoxazolyl, thiazolyl, pyridyl, pyridazinyl, pyrimidyl, quinolyl, isoquinolyl, indolyl, benzimidazolyl, quinazolyl, thionaphthenyl, chromonyl, thiochromonyl, chromenyl, thiochromenyl, benzoxadiazolyl or benzothiadiazolyl radical, it being possible for the radicals mentioned optionally to contain 1 to 3 identical or different substituents from the group comprising alkyl (1 to 15 carbon atoms), alkenyl (up to 15 carbon atoms), alkinyl (up to 15 C-atoms), alkoxy (1 to 15 C-atoms) fluorine, chlorine, bromine, iodine, trifluoromethyl, monofluoroalkoxy (1 to 5 C-atoms), polyfluoroalkoxy (1 to 5 C-atoms), hydroxyl, amino, monoalkylamino (1 to 5 C-atoms), dialkylamino (1 to 5 C-atoms), nitro, cyano, azido, carboxyl, carboxamido, sulphonamido, $SO_m$-alkyl (m = 0 to 2, 1 to 10 C-atoms), phenyl, benzyl, benzyloxy and benzylthio, it being possible for the last four substituents mentioned optionally to contain 1 to 3 radicals from the group comprising alkyl (1 to 4 C-atoms), alkoxy (1 to 4 C-atoms), alkylthio (1 to 4 C-atoms), fluorine, chlorine, cyano, nitro, hydroxyl, trifluoromethyl, amino, alkylamino (1 to 4 C-atoms) or dialkylamino (1 to 4 C-atoms),

$R^1$ represents a straight-chain, branched or cyclic saturated or unsaturated hydrocarbon radical (up to 15 C-atoms), which can optionally be interrupted in the chain by 1 or 2 non-adjacent oxygen or sulphur atoms and can optionally be substituted by one or more fluorine atoms, $HO_2$ trialkylsilyl (3 to 9 C-atoms), -OH, -Cl, -CN, amino, alkylamino (1 to 5 C-atoms) or dialkylamino (in each case 1 to 5 C-atoms),

$R^2$ represents hydrogen, -CHO, -CN or a straight-chain or branched, saturated or unsaturated hydrocarbon radical (up to 6 C-atoms),

$R^3$

represents hydrogen or a straight-chain or branched alkyl radical (up to 6 C-atoms), which is optionally interrupted in the alkyl chain by one or two non-adjacent oxygen atoms and can optionally be substituted by fluorine, chlorine, -CN, -OH or morpholino, and

$R^4$ represents hydrogen or a straight-chain or branched alkyl radical (up to 6 C-atoms).

3. Compounds of the general formula (I) according to Claim 1,

in which

R represents a phenyl, naphthyl, thienyl, oxazolyl, isoxazolyl, thiazolyl, pyridyl, pyrimidyl, quinolyl, isoquinolyl, indolyl, benzimidazolyl, quinazolyl, thionaphthenyl, chromonyl, thiochromonyl, chromenyl, thiochromenyl, benzoxadiazolyl or benzothiadiazolyl radical, it being possible for the radicals mentioned optionally to contain 1 or 2 identical or different substituents from the group comprising alkyl (1 to 10

carbon atoms), alkenyl (up to 10 carbon atoms), alkoxy (1 to 10 C-atoms), fluorine, chlorine, bromine, trifluoromethyl, monofluoroalkoxy (1 to 5 C atoms), polyfluoroalkoxy (1 to 5 C-atoms), hydroxyl, monoalkylamino (1 to 5 C-atoms), dialkylamino (1 to 5 C-atoms), nitro, cyano, azido, $SO_m$-alkyl (m = 0 to 2, 1 to 5 C-atoms), phenyl, benzyl, benzyloxy and benzylthio, it being possible for the last four substituents mentioned optionally to contain 1 to 3 radicals from the group comprising alkyl (1 to 3 C-atoms), alkoxy (1 to 3 C-atoms), alkylthio (1 to 3 C-atoms), fluorine, chlorine, cyano, nitro and trifluoromethyl,

$R^1$ represents a straight-chain, branched or cyclic, saturated or unsaturated hydrocarbon radical (up to 10 C-atoms), which can optionally be interrupted in the chain by 1 or 2 non-adjacent oxygen or sulphur atoms and can optionally be substituted by one or more fluorine atoms, $NO_2$, trialkylsilyl (3 to 6 C-atoms), -OH, -Cl, -CN, amino, alkylamino (1 to 3 C-atoms) or dialkylamino (in each case 1 to 3 C-atoms),

$R^2$ represents hydrogen or a straight-chain or branched, saturated or unsaturated hydrocarbon radical (up to 4 C-atoms),

$R^3$ represents hydrogen or a straight-chain or branched alkyl radical (up to 4 C-atoms), which is optionally interrupted in the alkyl chain by 1 or 2 non-adjacent oxygen atoms and can optionally be substituted by fluorine, chlorine, -OH or morpholino, and

$R^4$ represents hydrogen or a straight-chain alkyl radical (1 to 4 C-atoms).

4. Process for the preparation of compounds of the general formula (I)

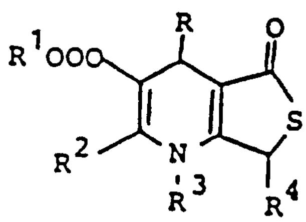

(I)

in which

R represents a phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl, quinoxalyl, thionaphthenyl, isothionaphthenyl, chromonyl, thiochromonyl, chromenyl, thiochromenyl, benzoxadiazolyl or benzothiadiazolyl radical, it being possible for the radicals mentioned optionally to contain 1 to 3 identical or different substituents from the group comprising alkyl (up to 20 carbon atoms), alkenyl (up to 20 carbon atoms), alkinyl (up to 20 C-atoms), alkoxy (1 to 20 C-atoms), fluorine, chlorine, bromine, iodine, trifluoromethyl, monofluoroalkoxy (1 to 10 C-atoms), polyfluoroalkoxy (1 to 10 C-atoms), hydroxyl, amino, monoalkylamino (1 to 10 C-atoms), dialkylamino (1 to 10 C-atoms), nitro, cyano, azido, carboxyl, carbalkoxy, carboxamido, sulphonamido, $SO_m$-alkyl (m = 0 to 2, 1 to 20 C-atoms), phenyl, benzyl, benzyloxy and benzylthio, it being possible for the last four substituents mentioned optionally to contain 1 to 3 radicals from the group comprising alkyl (1 to 5 C-atoms), alkoxy (1 to 5 C-atoms), alkylthio (1 to 5 C-atoms), fluorine, chlorine, bromine, iodine, cyano, nitro, azido, hydroxyl, trifluoromethyl, amino, alkylamino (1 to 6 C-atoms) or dialkylamino (in each case 1 to 6 C-atoms),

$R^1$ represents a straght-chain, branched or cyclic saturated or unsaturated hydrocarbon radical (up to 20 C-atoms), which can optionally be interrupted in the chain by 1 or 2 non-adjacent oxygen or sulphur atoms and which can optionally be substituted by one or more fluorine atoms, $NO_2$ trialkylsilyl (3 to 12 C-atoms), -OH, -Cl, Br, I, -CH, amino, alkylamino (1 to 10 C-atoms), dialkylamino (in each case 1 to 10 C-atoms), benzylalkylamino (alkyl radical with 1 to 8 C-atoms), phenyl, naphthyl or pyridyl,

$R^2$ represents hydrogen, $-NH_2$, -CHO, -CN, $CH_2OH$ or a straight-chain or branched, saturated or unsaturated hydrocarbon radical (up to 8 C-atoms),

$R^3$ represents hydrogen or a straight-chain or branched alkyl radical (up to 10 C-atoms), which is optionally interrupted in the alkyl chain by one or two nonadjacent oxygen atoms and can optionally be substituted by fluorine, chlorine, bromine, iodine, -CN, $-NH_2$, -OH or morpholino, and

$R^4$ represents hydrogen or a straight-chain or branched alkyl radical (up to 10 C-atoms) and their pharmaceuticalty acceptable salts, characterised in that benzylidene compounds of the general formula (II)

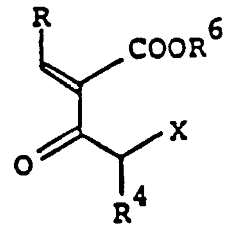

(II)

in which

R and $R^4$ have the abovementioned meaning,

X represents halogen and

$R^6$ represents an alkyl radical with up to 6 carbon atoms,

are reacted with thiolates of the general formula (III)

Me-S-$R^5$ (III)

in which

Me denotes an alkali metal and

$R^5$ represents a thiol-protective group

in the presence of inert organic solvents at temperatures of -60 to +80°C, alkali metal halide (MeX) being split off, to give compounds of the general formula (IV)

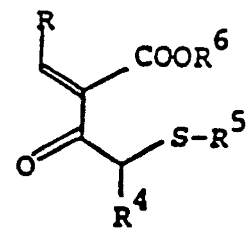

(IV)

in which

R, $R^4$, $R^5$ and $R^6$ have the abovementioned meaning,

and the compound of the formula (IV) is then reacted with aminocrotonic acid esters of the general formula (V)

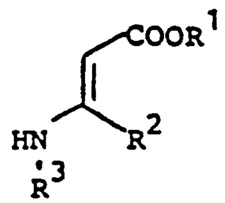

(V)

in which

$R^1$, $R^2$ and $R^3$ have the abovementioned meaning, in the presence of inert organic solvents to give the dihydropyridine of the general formula (VI)

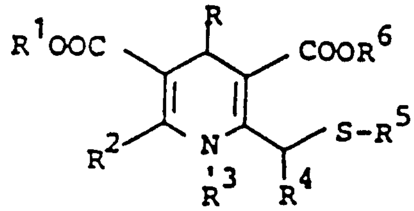

(VI)

in which

R, $R^1$ $R^2$ $R^3$ $R^4$ $R^5$ and $R^6$ have the abovementioned meaning, and this dihydropyridine of the formula (VI) is then lactonised, the thiol-protective group ($R^5$) being split off, to give compounds of the general formula (I).

5. Process according to Claim 4, characterised in that the reaction is carried out with compounds (IV) and (VI) being isolated.

6. Medicaments containing at least one compound of the general formula (I) according to Claim 1.

7. Process for the preparation of medicaments, characterised in that compounds of the general formula (I) according to Claim 1 are converted into a suitable administration form, if appropriate using inert auxiliaries and excipients.

8. Use of compounds of the general formula (I) according to Claim 1 to prepare medicaments for combating circulatory diseases.

9. Use of compounds of the general formula (I) according to Claim 1 to prepare medicaments for treating coronary diseases, hypertension, diabetes and disorders in the salt balance.

**Revendications**

1. Tétrahydrothiénopyridines de formule générale I

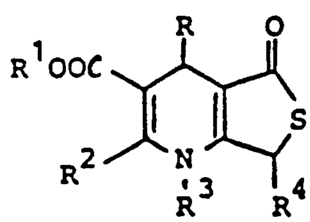

(I)

dans laquelle

R représente un groupe phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinoléyle, isoquinoléyle, indolyle, benzimidazolyle, quinazolyle, quinoxalyle, thionaphténtyle, isothionaphtenyle, chromonyle, thiochromonyle, chroményle, thiochroményle, benzoxadiazolyle ou benzothiadiazolyle, ces groupes pouvant le cas échéant porter un à trois substituants identiques ou différents choisis parmi les groupes alkyle (contenant 1 à 20 atomes de carbone), alcényle (contenant jusqu'à 20 atomes de carbone), alcynyle (contenant jusqu'à 20 atomes de carbone), alcoxy (contenant 1 à 20 atomes de carbone), le fluor, le chlore, le brome, l'iode, les groupes trifluorométhyle, monofluoralcoxy (contenant 1 à 10 atomes de carbone), polyfluoralcoxy (contenant 1 à 10 atomes de carbone), hydroxy, amino, monoalkylamino (contenant 1 à 10 atomes de carbone), dialkylamino (contenant 1 à 10 atomes de carbone), nitro, cyano, azido) carboxy, carboxamido, sulfonamido, $SO_m$-alkyle (m = 0 à 2, contenant 1 à 20 atomes de carbone), phényle, benzyle, benzyloxy ou benzylthio, les quatre substituants mentionnés en dernier pouvant eux-mêmes le cas échéant contenir 1 à 3 substituants choisis parmi les groupes alkyle (en $C_1$-$C_5$), alcoxy (contenant 1 à 5 atomes de carbone), alkylthio (contenant 1 à 5 atomes de carbone), le fluor, le chlore, le brome, l'iode, les groupes cyano, nitro, azido, hydroxy, trifluorométhyle, amino, alkylamino (contenant 1 à 6 atomes de carbone), ou dialkylamino (contenant chacun 1 à 6 atomes de carbone),

$R^1$ représente un groupe hydrocarbone à chaîne droite, ramifiée ou cyclique, saturé ou insaturé (contenant jusqu'à 20 atomes de carbone) qui peut le cas échéant être interrompu dans la chaîne par 1 ou 2 atomes d'oxygène ou de soufre non voisins et qui peut le cas échéant porter 1 ou plusieurs substituants choisis parmi les atomes de fluor, des groupes $NO_2$, trialkylsilyle (contenant 3 à 12 atomes de carbone), -OH, le chlore, le brome, l'iode, les groupes -CN, amino, alkylamino (contenant 1 à 10 atomes de carbone), dialkylamino (contenant chacun 1 à 10 atomes de carbone), benzylalkylamino (avec un groupe alkyle qui contient 1 à 8 atomes de carbone), phényle, naphtyle ou pyridyle,

$R^2$ représente l'hydrogène, un groupe $-NH_2$ -CHO, -CN, $CH_2OH$ ou un groupe hydrocarboné à chaîne droite ou ramifiée, saturé ou insaturé (contenant jusqu'à 8 atomes de carbone),

$R^3$ représente l'hydrogène ou un groupe alkyle à chaîne droite ou ramifiée (contenant 1 à 10 atomes de carbone), qui peut le cas échéant être interrompu dans la chaîne alkyle par 1 ou 2 atomes d'oxygène non voisins et qui peut le cas échéant être substitué par le fluor, le chlore, le brome, l'iode, des groupes -CN, $-NH_2$ -OH ou morpholino, et

$R^4$ représente l'hydrogène ou un groupe alkyle à chaîne droite ou ramifiée (contenant jusqu'à 10 atomes de carbone) et leurs sels acceptables pour l'usage pharmaceutique.

2. Composés de formule générale I selon la revendication 1, dans laquelle

R représente un groupe phényle, naphtyle, thiényle, pyrryle, midazolyle, oxazolyle, isoxazolyle, thiazolyle, pyridyle, pyridazinyle, pyrimidyle, quinoléyle, isoquinoléyle, indolyle, benzimidazolyle, quinazolyle, thionaphténylе, chromonyle, thiochromonyle, chroménylе, thiochroménylе, benzoxadiazolyle ou benzothiadiazolyle, les groupes mentionnés pouvant eux-mêmes porter le cas échéant un à trois substituants identiques ou différents choisis parmi les groupes alkyle (contenant 1 à 15 atomes de carbone), alcényle (contenant jusqu'à 15 atomes de carbone), alcynyle (contenant jusqu'à 15 atomes de carbone), alcoxy (contenant 1 à 15 atomes de carbone), le fluor, le chlore, le brome, l'iode, les groupes trifluorométhyle, monofluoralcoxy (contenant 1 à 5 atomes de carbone), polyfluoralcoxy (contenant 1 à 5 atomes de carbone), hydroxy-amino, monoalkylamino (contenant 1 à 5 atomes de carbone), dialkylamino (contenant 1 à 5 atomes de carbone), nitro, cyano, azido, carboxy, carboxamido, sulfonamido, $SO_m$-alkyle ($m = 0$ à 2, contenant 1 à 10 atomes de carbone), phényle, benzyle, benzyloxy ou benzylthio, les quatre substituants mentionnés en dernier pouvant eux-mêmes porter le cas échéant un à trois substituants choisis parmi les groupes alkyle (contenant 1 à 4 atomes de carbone), alcoxy (contenant 1 à 4 atomes de carbone), alkylthio (contenant 1 à 4 atomes de carbone), le fluor, le chlore, les groupes cyano, nitro, hydroxy, trifluorométhyle, amino, alkylamino (contenant 1 à 4 atomes de carbone) ou dialkylamino (contenant 1 à 4 atomes de carbone),

$R^1$ représente un groupe hydrocarboné à chaîne droite, ramifiée ou cyclique, saturé ou insaturé (contenant jusqu'à 15 atomes de carbone) qui peut le cas échéant être interrompu dans la chaîne par 1 ou 2 atomes d'oxygène ou de soufre non voisins et qui peut le cas échéant porter un ou plusieurs substituants choisis parmi les atomes de fluor, les groupes $NO_2$ trialkylsilyle (contenant 3 à 9 atomes de carbone), -OH, le chlore, les groupes -CN, amino, alkylamino (contenant 1 à 5 atomes de carbone) ou dialkylamino (contenant chacun 1 à 5 atomes de carbone),

$R^2$ représente l'hydrogène, un groupe -CHO, -CN ou un groupe hydrocarboné à chaîne droite ou ramifiée, saturé ou insaturé (contenant jusqu'à 6 atomes de carbone),

$R^3$ représente l'hydrogène ou un groupe alkyle à chaîne droite ou ramifiée (contenant jusqu'à 6 atomes de carbone) qui peut le cas échéant être interrompu dans la chaîne alkyle par 1 ou 2 atomes d'oxygène non voisins et qui peut le cas échéant être substitué par le fluor, le chlore, des groupes -CN, -OH ou morpholino, et

$R^4$ représente l'hydrogène ou un groupe alkyle à chaîne droit ou ramifiée (contenant jusqu'à 6 atomes de carbone)

3. Composés de formule générale 1 selon la revendication 1, dans laquelle

R représente un groupe phényle, naphtyle, thiényle, oxazolyle, isoxazolyle, thiazolyle, pyridyle, pyrimidyle, quinoléyle, isoquinoléyle, indolyle, benzimidazolyle, quinazolyle, thionaphténylе, chromonyle, thiochromonyle, chroménylе, thiochroménylе, benzoxadiazolyle ou benzothiadiazolyle, ces groupes pouvant le cas échéant porter 1 ou 2 substituants identiques ou différents choisis parmi les groupes alkyle (contenant 1 à 10 atomes de carbone), alcényle (contenant jusqu'à 10 atomes de carbone), alcoxy (contenant 1 à 10 atomes de carbone), le fluor, le chlore, le brome, des groupes trifluorométhyle, monofluoralcoxy (contenant 1 à 5 atomes de carbone), polyfluoralcoxy (contenant 1 à 5 atomes de

carbone), hydroxy, monoalkylamino (contenant 1 à 5 atomes de carbone), dialkylamino (contenant 1 à 5 atomes de carbone), nitro, cyano, azido, $SO_m$-alkyle (m 0 à 2, contenant 1 à 5 atomes de carbone), phényle, benzyle, benzyloxy, ou benzylthio, les quatre substituants mentionnés en dernier pouvant eux-mêmes le cas échéant porter 1 à 3 substituants choisis parmi les groupes alkyle (contenant 1 à 3 atomes de carbone), alcoxy (contenant 1 à 3 atomes de carbone), alkylthio (contenant 1 à 3 atomes de carbone), le fluor, le chlore, les groupes cyano, nitro, trifluorométhyle,

$R^1$ représente un groupe hydrocarboné à chaîne droite, ramifiée ou cyclique, saturé ou insaturé (contenant jusqu'à 10 atomes de carbone) qui peut le cas échéant être interrompu dans la chaîne alkyle par 1 ou 2 atomes d'oxygène ou de soufre non voisins et qui peut le cas échéant porter un ou plusieurs substituants choisis parmi les atomes de fluor, les groupes $NO_2$, trialkylsilyle (contenant 3 à 6 atomes de carbone), -OH, le chlore, les groupes -CN, amino, alkylamino (contenant 1 à 3 atomes de carbone), ou dialkylamino (contenant chacun 1 à 3 atomes de carbone),

$R^2$ représente l'hydrogène ou un groupe hydrocarboné à chaîne droite ou ramifiée, saturé ou insaturé (contenant jusqu'à 4 atomes de carbone),

$R^3$ représente l'hydrogène ou un groupe alkyle à chaîne droite ou ramifiée (contenant jusqu'à 4 atomes de carbone) qui peut le cas échant être interrompu dans la chaîne alkyle par 1 ou 2 atomes d'oxygène non voisins et qui peut le cas échéant être substitué par le fluor, le chlore, des groupes -OH ou morpholino, et

$R^4$

représente l'hydrogène ou un groupe alkyle à chaîne droite (contenant 1 à 4 atomes de carbone).

4. Procédé de préparation des composés de formule générale

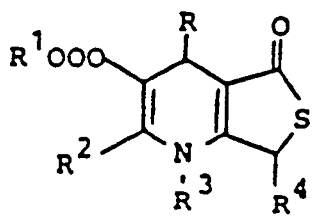

(I)

dans laquelle

R représente un groupe phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolélyle, isoquinolélyle, indolyle, benzimidazolyle, quinazolyle, quinoxalyle, thionaphtényle, isothionaphtényle, chromonyle, thiochromonyle, chroményle, thiochroményle, benzoxadiazolyle ou benzothiadiazolyle, les groupes mentionnés pouvant le cas échéant porter 1 à 3 substituants identiques ou différents choisis parmi les groupes alkyle (contenant 1 à 20 atomes de carbone), les groupes alcéyle (contenant jusqu'à 20 atomes de carbone), les groupes alcynyle (contenant jusqu'à 20 atomes de carbone), les groupes alcoxy (contenant 1 à 20 atomes de carbone), le fluor, le chlore, le brome, l'iode) les groupes trifluorométhyle, monofluoralcoxy (contenant 1 à 10 atomes de carbone), polyfluoralcoxy (contenant 1 à 10 atomes de carbone), hydroxy, amino, monoalkylamino (contenant 1 à 10 atomes de carbone), dialkylamino (contenant 1 à 10 atomes de carbone), nitro, cyano, azido, carboxy, carbalcoxy, carboxamido, sulfonamido, $SO_m$-alkyle (m = 0 à 2, contenant 1 à 20 atomes de carbone), phényle, benzyle, benzyloxy ou benzylthio, les quatre substituants mentionnés en dernier pouvant eux-mêmes le cas échéant porter 1 à 3 substituants choisis parmi les groupes alkyle (contenant 1 à 5 atomes de carbone), alcoxy (contenant 1 à 5 atomes de carbone), alkylthio (contenant 1 à 5 atomes de carbone), le fluor, le chlore, le brome, l'iode, des groupes cyano, nitro, azido, hydroxy, trifluorométhyle, amino, alkylamino (contenant 1 à 6 atomes de carbone) ou dialkylamino (contenant chacun 1 à 6 atomes de carbone),

$R^1$ représente un groupe hydrocarboné à chaîne droite) ramifiée ou cyclique, saturé ou insaturé (contenant jusqu'à 20 atomes de carbone) qui peut le cas échéant être interrompu dans la chaîne par 1 ou 2 atomes d'oxygène ou de soufre non voisins et qui peut le cas échéant porter un ou plusieurs substituants choisis parmi les atomes de fluor, les groupes $NO_2$, trialkylsilyle (contenant 3 à 12 atomes de carbone), -OH, le chlore, le brome, l'iode) les groupes -CN, amino, alkylamino (contenant 1 à 10 atomes de carbone), dialkylamino (contenant chacun 1 à 10 atomes de carbone), benzylalkylamino (avec un groupe alkyle qui contient 1 à 8 atomes de carbone), phényle, naphtyle ou pyridyle,

$R^2$ représente l'hydrogène, un groupe $NH_2$ -CHO, -CN, $CH_2OH$ ou un groupe hydrocarboné à chaîne droite ou

ramifiée, saturé ou insaturé (contenant jusqu'à 8 atomes de carbone),

$R^3$ représente l'hydrogène ou un groupe alkyle à chaîne droite ou ramifiée (contenant jusqu'à 10 atomes de carbone) qui peut le cas échéant être interrompu dans la chaîne alkyle par 1 ou 2 atomes d'oxygène non voisins et qui peut le cas échéant être substitué par le fluor, le chlore, le brome, l'iode, des groupes -CN, $-NH_2$) -OH ou morpholino, et

$R^4$ représente l'hydrogène ou un groupe alkyle à chaîne droite ou ramifiée (contenant jusqu'à 10 atomes de carbone),

et de leurs sels acceptables pour l'usage pharmaceutique) caractérisé en ce que l'on fait réagir des composés benzylidéniques de formule générale II

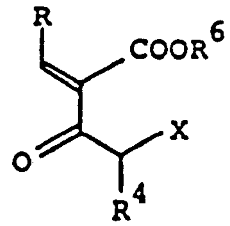

(II)

dans laquelle R et $R^4$ ont les significations indiquées ci-dessus,

X représente un halogène, et

$R^6$ représente un groupe alkyle contenant jusqu'à 6 atomes de carbone, avec des thiolates de formule générale III

$Me-S-R^5$ (III)

dans laquelle

Me représente un métal alcalin, et

$R^5$ représente un groupe protecteur du groupe thiol, en présence de solvants organiques inertes, à des températures allant de -60 jusqu'à +80°C, avec séparation d'un halogénure alcalin (MeX), ce qui donne des composés de formule générale IV

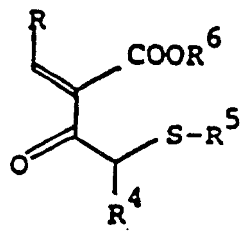

(IV)

dans laquelle

R, $R^4$, $R^5$ et $R^6$ ont les significations indiquées ci-dessus, qu'on fait réagir avec des esters aminocrotoniques de formule générale V

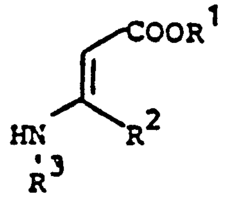

(V)

dans laquelle

$R^1$, $R^2$ et $R^3$ ont les significations indiquées ci-dessus, en présence de solvants organiques inertes, ce qui

donne une dihydropyridine de formule générale VI

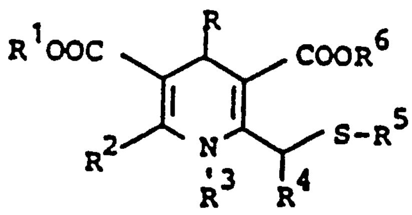

(VI)

dans laquelle

R, $R^1$, $R^2$, $R^3$, $R^4$, $R^5$, $R^6$ ont les significations indiquées ci-dessus,

qu'on lactonise en composés de formule générale I avec séparation du groupe protecteur du groupe thiol ($R^5$).

5. Procédé selon la revendication 4, caractérisé en ce que la réaction est effectuée avec isolement des composés IV et VI.

6. Médicament contenant au moins un composé de formule générale I selon la revendication 1.

7. Procédé de préparation de médicaments, caractérisé en ce que l'on met des composés de formule générale I selon la revendication 1 sous une forme d'administration appropriée éventuellement avec utilisation de produits auxiliaires et véhicules inertes.

8. Utilisation des composés de formule générale 1 selon la revendication 1, pour la préparation de médicaments pour le traitement des maladies circulatoires.

9. Utilisation des composés de formule générale 1 selon la revendication 1, pour la préparation de médicaments pour le traitement des maladies coronariennes, de l'hypertonie, du diabète et des troubles du métabolisme des sels.